# EUROPEAN PATENT APPLICATION

(11) **EP 2 246 334 A1**
(43) Date of publication of application: **03.11.2010**
(21) Application number: 09703765.9
(22) Date of filing: 23.01.2009
(51) Int. Cl.: C07D 235/12, A61K 31/4184, A61P 25/28, A61P 43/00

(54) **MEDICINAL COMPOSITION FOR INHIBITING AMYLOID- PROTEIN DEPOSITION**

(30) Priority: 25.01.2008 JP 2008014914
(71) Applicant: Sumitomo Chemical Company, Limited, Tokyo 104-8260 (JP)
(72) Inventor: MOTONAGA, Kozo, Ibaraki-shi Osaka 567-0841 (JP); SAITO, Koichi, Osaka-shi Osaka (JP); HARADA, Koichiro, Toyonaka-shi Osaka (JP)
(74) Representative: Duckworth, Timothy John
(86) International application number: PCT/JP2009/051522
(87) International publication number: WO 2009/093761

(57) **Abstract**

The present invention provides: a pharmaceutical composition for inhibiting amyloid-β protein accumulation comprising a compound of the formula (I) or a pharmaceutically acceptable salt thereof as an active ingredient; a method for inhibiting amyloid-β protein accumulation, comprising a step of administering an effective amount of the compound of the formula (I) or a pharmaceutically acceptable salt thereof to a mammal which may be diagnosed with an amyloid-β protein-related disease; and so on.

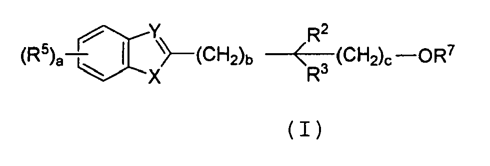

## Description

### Technical Field

The present invention relates to a pharmaceutical composition that inhibits amyloid-β protein accumulation, and so on.

### Background Art

An amyloid-β protein (hereinafter, sometimes referred to as Aβ) is an insoluble protein consisting of approximately 40 amino acid residues, and is generated by cleavage of an amyloid-β precursor protein by β-secretase and γ-secretase. The β-secretase has been identified as an aspartic protease (Neuron, 27, 419-422, 2000). It has been revealed that a gene responsible for familial Alzheimer's disease (FAD) and presenilin or a complex containing presenilin are involved in expression of a γ-secretase activity (Neuron, 27, 419-422, 2000).

It has been known that accumulation of the Aβ in each organ and tissue in a living body causes functional impairment in the organ and tissue. Diseases in which each organ and tissue is impaired by accumulation of the Aβ are collectively called Aβ-related diseases (amyloidosis).

### Disclosure of the Invention

Based on the foregoing findings, a compound that inhibits Aβ accumulation is useful as a medicine capable of treating or preventing an Aβ-related disease. An object of the present invention is to provide a compound that inhibits Aβ accumulation.

The present invention includes the following inventions.

### [Invention 1]

A pharmaceutical composition for inhibiting amyloid-β protein accumulation comprising as an active ingredient a compound of the following formula (I): 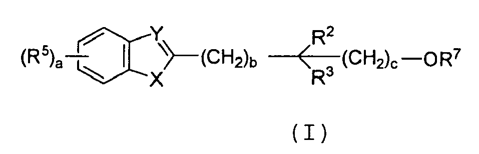 or a pharmaceutically acceptable salt thereof,
in the formula (I):
X is O, S or NR¹;
Y is N or CR⁴;
R¹ is hydrogen, C1-C6 alkyl, fluorinated C1-C6 alkyl, C1-C6 alkylsulfonyl, benzenesulfonyl, toluenesulfonyl, cyano C1-C6 alkyl, (C1-C6 alkoxy)carbonyl(C1-C6 alkyl), (C1-C6 alkoxy)alkyl or -(C1-C6 alkyl)-S(O)_{d}(C1-C6 alkyl);
R⁴ is hydrogen, halogen, C1-C6 alkyl, C2-C6 alkenyl, C2-C6 alkynyl or cyano, wherein C1-C6 alkyl, C2-C6 alkenyl or C2-C6 alkynyl may be substituted with tri(C1-C6 alkyl)silyl on a terminal carbon atom;
a is any one of integers of 0 to 4;
R⁵ is selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino, di(C1-C6 alkyl)amino, (C1-C6 alkyl)carbonyl, (C1-C6 alkoxy)carbonyl, -C(O)-N(R^{A})₂, -S(O)_{d}-(C1-C6alkyl), -SO₂-N(R^{A})₂, -N(R^{A})-C(O)-(C1-C6 alkyl), -N(R^{A})-C(O)-(halogen-substituted C1-C6 alkyl) and aryl;
wherein R^{A} in each occurrence independently represents hydrogen or C1-C6 alkyl;
wherein, optionally, aryl may be substituted with one or more substituents independently selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino and di(C1-C6 alkyl)amino;
b is 0 or 1;
c is 0 or 1;
R⁷ is selected from the group consisting of hydrogen, C1-C6 alkyl and tri(C1-C6 alkyl)silyl;
R² is selected from the group consisting of hydrogen, C1-C6 alkyl, halogen-substituted C1-C6 alkyl and -(CH₂)ₑ-Z-R⁶;
R³ is selected from the group consisting of C1-C6 alkyl, halogen-substituted C1-C6 alkyl and -(CH₂)ₑ-Z-R⁶;
wherein Z in each occurrence is independently selected from the group consisting of -S(O)_{d}-, -O-, -O-C(O)-, -NH- and -N(C1-C6 alkyl)-;
wherein R⁶ in each occurrence is independently selected from the group consisting of C1-C6 alkyl, halogen-substituted C1-C6 alkyl, C2-C6 alkenyl, aryl, aralkyl, biphenyl, C3-C7 cycloalkyl, C3-C7 cycloalkyl-(C1-C6 alkyl), heteroaryl and heteroaryl-(C1-C6 alkyl);
wherein a cycloalkyl, aryl or heteroaryl group, whether alone or as a part of a substituent group, may be substituted with one or more substituents independently selected from the group consisting of halogen, hydroxy, carboxy, C1-C6 alkyl, halogen-substituted C1-C6 alkyl, C1-C6 alkoxy, cyano, nitro, amino, C1-C6 alkylamino, di(C1-C6 alkyl)amino, -S(O)_{d}-(C1-C6 alkyl) and SO₂-N(R^{A})₂; provided that, when Z is O, NH or N(C1-C6 alkyl), R⁶ is selected from the group consisting of C1-C6alkyl, halogen-substituted C1-C6 alkyl, aryl, aralkyl, biphenyl, C3-C7 cycloalkyl, C3-C7 cycloalkyl-(C1-C6 alkyl), heteroaryl and heteroaryl-(C1-C6 alkyl);
provided that, when R² is methyl, R³ is selected from the group consisting of C2-C6 alkyl, halogen-substituted C1-C6 alkyl and -(CH₂)ₑ-Z-R⁶;
provided that, when X is NR¹, R¹ is hydrogen or C1-C6 alkyl, b is 1, c is 0, R⁴ is hydrogen, R⁷ is hydrogen, a is 0 and when R² is CF₃, R³ is selected from the group consisting of C1-C6 alkyl, halogen-substituted C1-C6 alkyl except CF₃, and -(CH₂)ₑ-Z-R⁶; provided that, when X is NH, R⁴ is methyl, b is 0, c is 0, R⁷ is hydrogen, a is 0 and when R² is methyl, R³ is selected from the group consisting of C1-C6 alkyl, halogen-substituted C1-C6 alkyl except CF₃, and -(CH₂)ₑ-Z-R⁶;
provided that, when X is NR¹, R¹ is hydrogen or C1-C6 alkyl, R⁴ is hydrogen or methyl, b is 0, c is 0, R⁷ is hydrogen, a is 0 and when R² is hydrogen or methyl, R³ is selected from the group consisting of C1-C6 alkyl, halogen-substituted C1-C6 alkyl and -(CH₂)ₑ-Z-R⁶, excluding -(CH₂)_{f}-N(R^{A})-(C1-C6 alkyl) or -(CH₂)₃-N(R^{A})-(benzyl);
provided that, when X is NH, R⁴ is hydrogen, b is 1, c is 0, R⁷ is hydrogen, a is 0 and when R² is hydrogen, R³ is selected from the group consisting of C1-C6 alkyl, halogen-substituted C1-C6 alkyl and -(CH₂)ₑ-Z-R⁶, excluding -(CH₂)-NH-(C1-C6 alkyl),
provided that, when X is NH, R⁴ is hydrogen, a is 0, R⁷ is hydrogen, b is 0, c is 0 and when R² is CF₃, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NH, R⁴ is hydrogen, a is 0, R⁷ is hydrogen, b is 1, c is 1 and when R² is hydrogen, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NR¹ , R¹ is hydrogen or methyl, R⁴ is hydrogen or methyl, b is 0, c is 0, a is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding -CH₂-O-C1-C6 alkyl or -CH₂-O-benzyl;
provided that, when X is O, R⁴ is hydrogen, b is 0, c is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding CH₂-O-phenyl;
wherein, optionally, phenyl may be independently substituted with one to two substituents selected from among C1-C6 alkyl, hydroxy-substituted C1-C6 alkyl, carboxy and -C(O)-(C1-C6 alkoxy); d is any one of integers of 0 to 2;
e is any one of integers of 1 to 4; and
f is 1 or 2.

### [Invention 2]

An amyloid-β protein accumulation-inhibitory agent comprising as an active ingredient a compound of the following formula (I): 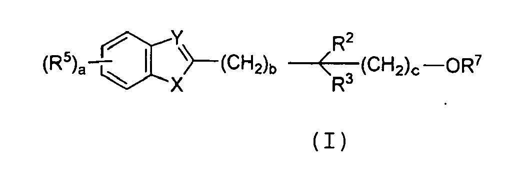 or a pharmaceutically acceptable salt thereof,
in the formula (I):
X is O, S or NR¹;
Y is N or CR⁴;
R¹ is hydrogen, C1-C6 alkyl, fluorinated C1-C6 alkyl, C1-C6 alkylsulfonyl, benzenesulfonyl, toluenesulfonyl, cyano C1-C6 alkyl, (C1-C6 alkoxy) carbonyl (C1-C6 alkyl), (C1-C6 alkoxy) alkyl or -(C1-C6 alkyl)-S(O)_{d}(C1-C6 alkyl);
R⁴ is hydrogen, halogen, C1-C6alkyl, C2-C6 alkenyl, C2-C6 alkynyl or cyano, wherein C1-C6 alkyl, C2-C6 alkenyl or C2-C6 alkynyl may be substituted with tri(C1-C6 alkyl)silyl on a terminal carbon atom;
a is any one of integers of 0 to 4;
R⁵ is selected from the group consisting of halogen, -OH, carboxy,
C1-C6 alkyl, halogen-substituted C1-C6 alkyl, C1-C6 alkoxy, halogen-substituted C1-C6 alkoxy, cyano, nitro, amino, C1-C6 alkylamino, di(C1-C6 alkyl)amino, (C1-C6 alkyl)carbonyl, (C1-C6 alkoxy) carbonyl, -C(O)-N(R^{A})₂, -S(O)_{d}-(C1-C6 alkyl) , -SO₂-N (R^{A})₂, -N(R^{A})-C(O)-(C1-C6 alkyl), -N(R^{A})-C(O)-(halogen-substituted C1-C6 alkyl) and aryl;
wherein R^{A} in each occurrence independently represents hydrogen or C1-C6 alkyl;
wherein, optionally, aryl may be substituted with one or more substituents independently selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino and di(C1-C6 alkyl)amino;
b is 0 or 1;
c is 0 or 1;
R⁷ is selected from the group consisting of hydrogen, C1-C6 alkyl and tri(C1-C6 alkyl)silyl;
R² is selected from the group consisting of hydrogen, C1-C6 alkyl, halogen-substituted C1-C6 alkyl and -(CH₂)ₑ-Z-R⁶;
R³ is selected from the group consisting of C1-C6 alkyl, halogen-substituted C1-C6 alkyl and -(CH₂)ₑ-Z-R⁶;
wherein Z in each occurrence is independently selected from the group consisting of -S(O)_{d}-, -O-, -O-C(O)-, -NH- and -N(C1-C6 alkyl)-;
wherein R⁶ in each occurrence is independently selected from the group consisting of C1-C6 alkyl, halogen-substituted C1-C6 alkyl, C2-C6 alkenyl, aryl, aralkyl, biphenyl, C3-C7 cycloalkyl, C3-C7 cycloalkyl-(C1-C6 alkyl), heteroaryl and heteroaryl-(C1-C6 alkyl);
wherein a cycloalkyl, aryl or heteroaryl group, whether alone or as a part of a substituent group, may be substituted with one or more substituents independently selected from the group consisting of halogen, hydroxy, carboxy, C1-C6 alkyl, halogen-substituted C1-C6 alkyl, C1-C6 alkoxy, cyano, nitro, amino, C1-C6 alkylamino, di(C1-C6 alkyl)amino, -S(O)_{d}-(C1-C6 alkyl) and -SO₂-N(R^{A})₂; provided that, when Z is O, NH or N(C1-C6 alkyl), R⁶ is selected from the group consisting of C1-C6 alkyl, halogen-substituted C1-C6 alkyl, aryl, aralkyl, biphenyl, C3-C7 cycloalkyl, C3-C7 cycloalkyl-(C1-C6 alkyl), heteroaryl and heteroaryl-(C1-C6 alkyl);
provided that, when R² is methyl, R³ is selected from the group consisting of C2-C6 alkyl, halogen-substituted C1-C6 alkyl and -(CH₂)ₑ-Z-R⁶;
provided that, when X is NR¹, R¹ is hydrogen or C1-C6 alkyl, b is 1, c is 0, R⁴ is hydrogen, R⁷ is hydrogen, a is 0 and when R² is CF₃, R³ is selected from the group consisting of C1-C6 alkyl, halogen-substituted C1-C6 alkyl except CF₃, and -(CH₂)ₑ-Z-R⁶; provided that, when X is NH, R⁴ is methyl, b is 0, c is 0, R⁷ is hydrogen, a is 0 and when R² is methyl, R³ is selected from the group consisting of C1-C6 alkyl, halogen-substituted C1-C6 alkyl except CF₃, and -(CH₂)ₑ-Z-R⁶;
provided that, when X is NR¹, R¹ is hydrogen or C1-C6 alkyl, R⁴ is hydrogen or methyl, b is 0, c is 0, R⁷ is hydrogen, a is 0 and when R² is hydrogen or methyl, R³ is selected from the group consisting of C1-C6 alkyl, halogen-substituted C1-C6 alkyl and -(CH₂)ₑ-Z-R⁶, excluding -(CH₂)_{f}-N(R^{A})-(C1-C6 alkyl) or -(CH₂)₃-N(R^{A})-(benzyl);
provided that, when X is NH, R⁴ is hydrogen, b is 1, c is 0, R⁷ is hydrogen, a is 0 and when R² is hydrogen, R³ is selected from the group consisting of C1-C6 alkyl, halogen-substituted C1-C6 alkyl and -(CH₂)ₑ-Z-R⁶, excluding -(CH₂)-NH-(C1-C6 alkyl), provided that, when X is NH, R⁹ is hydrogen, a is 0, R⁷ is hydrogen, b is 0, c is 0 and when R² is CF₃, R³ is selected from the group consisting of C1-C6 alkyl, halogen-substituted C1-C6 alkyl and -(CH₂)ₑ-Z-R⁶, excluding -CH₂-O-C(O)-CH₃;
   provided that, when X is NH, R⁴ is hydrogen, a is 0, R⁷ is hydrogen, b is 1, c is 1 and when R² is hydrogen, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NR¹, R¹ is hydrogen or methyl, R⁴ is hydrogen or methyl, b is 0, c is 0, a is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding -CH₂-O-C1-C6 alkyl or -CH₂-O-benzyl;
provided that, when X is O, R⁴ is hydrogen, b is 0, c is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding CH₂-O-phenyl;
wherein, optionally, phenyl may be independently substituted with one to two substituents selected from among C1-C6 alkyl, hydroxy-substituted C1-C6 alkyl, carboxy and -C(O)-(C1-C6 alkoxy); d is any one of integers of 0 to 2;
e is any one of integers of 1 to 4; and
f is 1 or 2.

### [Invention 3]

Use of a compound of the following formula (I): 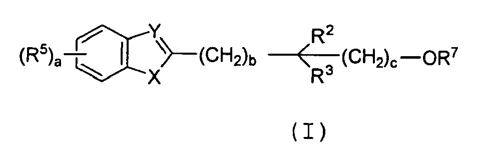 or a pharmaceutically acceptable salt thereof for inhibiting amyloid-β protein accumulation,
in the formula (I):
X is O, S or NR¹;
Y is N or CR⁴;
R¹ is hydrogen, C1-C6 alkyl, fluorinated C1-C6 alkyl, C1-C6 alkylsulfonyl, benzenesulfonyl, toluenesulfonyl, cyano C1-C6 alkyl, (C1-C6 alkoxy)carbonyl(C1-C6 alkyl), (C1-C6 alkoxy)alkyl or -(C1-C6 alkyl)-S(O)_{d}(C1-C6 alkyl);
R⁴ is hydrogen, halogen, C1-C6 alkyl, C2-C6 alkenyl, C2-C6 alkynyl or cyano, wherein C1-C6 alkyl, C2-C6 alkenyl or C2-C6 alkynyl may be substituted with tri(C1-C6 alkyl)silyl on a terminal carbon atom;
a is any one of integers of 0 to 4;
R⁵ is selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino, di(C1-C6 alkyl)amino, (C1-C6 alkyl)carbonyl, (C1-C6 alkoxy) carbonyl, -C(O)-N(R^{A})₂, -S(O)_{d}-(C1-C6 alkyl), -SO₂-N(R^{A})₂, -N(R^{A})-C(O)-(C1-C6 alkyl), -N(R^{A})-C(O)-(halogen-substituted C1-C6 alkyl) and aryl;
wherein R^{A} in each occurrence independently represents hydrogen or C1-C6 alkyl;
wherein, optionally, aryl may be substituted with one or more substituents independently selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino and di(C1-C6 alkyl)amino;
b is 0 or 1;
c is 0 or 1;
R⁷ is selected from the group consisting of hydrogen, C1-C6 alkyl and tri(C1-C6 alkyl)silyl;
R² is selected from the group consisting of hydrogen, C1-C6 alkyl, halogen-substituted C1-C6 alkyl and -(CH₂)ₑ-Z-R⁶;
R³ is selected from the group consisting of C1-C6 alkyl, halogen-substituted C1-C6 alkyl and -(CH₂)ₑ-Z-R⁶;
wherein Z in each occurrence is independently selected from the group consisting of -S(O)_{d}-, -O-, -O-C(O)-, -NH- and -N(C1-C6 alkyl)-;
wherein R⁶ in each occurrence is independently selected from the group consisting of C1-C6 alkyl, halogen-substituted C1-C6 alkyl, C2-C6 alkenyl, aryl, aralkyl, biphenyl, C3-C7 cycloalkyl, C3-C7 cycloalkyl-(C1-C6 alkyl), heteroaryl and heteroaryl-(C1-C6 alkyl);
wherein a cycloalkyl, aryl or heteroaryl group, whether alone or as a part of a substituent group, may be substituted with one or more substituents independently selected from the group consisting of halogen, hydroxy, carboxy, C1-C6 alkyl, halogen-substituted C1-C6 alkyl, C1-C6 alkoxy, cyano, nitro, amino, C1-C6 alkylamino, di(C1-C6 alkyl)amino, -S(O)_{d}-(C1-C6 alkyl) and -SO₂-N(R^{A})₂;
provided that, when Z is O, NH or N(C1-C6 alkyl), R⁶ is selected from the group consisting of C1-C6alkyl, halogen-substituted C1-C6 alkyl, aryl, aralkyl, biphenyl, C3-C7 cycloalkyl, C3-C7 cycloalkyl-(C1-C6 alkyl), heteroaryl and heteroaryl-(C1-C6 alkyl);
provided that, when R² is methyl, R³ is selected from the group consisting of C2-C6 alkyl, halogen-substituted C1-C6 alkyl and -(CH₂)ₑ-Z-R⁶;
provided that, when X is NR¹, R¹ is hydrogen or C1-C6 alkyl, b is 1, c is 0, R⁴ is hydrogen, R⁷ is hydrogen, a is 0 and when R² is CF₃, R³ is selected from the group consisting of C1-C6 alkyl, halogen-substituted C1-C6 alkyl except CF₃, and -(CH₂)ₑ-Z-R⁶;
provided that, when X is NH, R⁴ is methyl, b is 0, c is 0, R⁷ is hydrogen, a is 0 and when R² is methyl, R³ is selected from the group consisting of C1-C6 alkyl, halogen-substituted C1-C6 alkyl except CF₃, and -(CH₂)ₑ-Z-R⁶;
provided that, when X is NR¹, R¹ is hydrogen or C1-C6 alkyl, R⁴ is hydrogen or methyl, b is 0, c is 0, R⁷ is hydrogen, a is 0 and when R² is hydrogen or methyl, R³ is selected from the group consisting of C1-C6 alkyl, halogen-substituted C1-C6 alkyl and -(CH₂)ₑ-Z-R⁶, excluding -(CH₂)_{f}-N(R^{A})-(C1-C6 alkyl) or -(CH₂)₃-N(R^{A})-(benzyl);
provided that, when X is NH, R⁴ is hydrogen, b is 1, c is 0, R⁷ is hydrogen, a is 0 and when R² is hydrogen, R³ is selected from the group consisting of C1-C6 alkyl, halogen-substituted C1-C6 alkyl and -(CH₂)ₑ-Z-R⁶, excluding -(CH₂)-NH-(C1-C6 alkyl), provided that, when X is NH, R⁴ is hydrogen, a is 0, R⁷ is hydrogen, b is 0, c is 0 and when R² is CF₃, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NH, R⁴ is hydrogen, a is 0, R⁷ is hydrogen, b is 1, c is 1 and when R² is hydrogen, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NR¹, R¹ is hydrogen or methyl, R⁴ is hydrogen or methyl, b is 0, c is 0, a is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding -CH₂-O-C1-C6 alkyl or -CH₂-O-benzyl;
provided that, when X is O, R⁴ is hydrogen, b is 0, c is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding CH₂-O-phenyl;
wherein, optionally, phenyl may be independently substituted with one to two substituents selected from among C1-C6 alkyl, hydroxy-substituted C1-C6 alkyl, carboxy and -C(O)-(C1-C6 alkoxy); d is any one of integers of 0 to 2;
e is any one of integers of 1 to 4; and
f is 1 or 2.

### [Invention 4]

A method for inhibiting amyloid-β protein accumulation comprising administering an effective amount of a compound of the following formula (I): 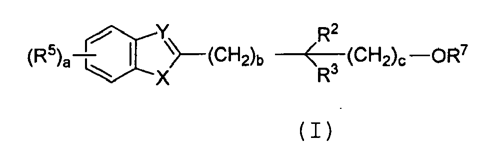 or a pharmaceutically acceptable salt thereof to a mammal which may be diagnosed with an amyloid-β protein-related disease,
in the formula (I):
X is O, S or NR¹;
Y is N or CR⁴;
R¹ is hydrogen, C1-C6 alkyl, fluorinated C1-C6 alkyl, C1-C6 alkylsulfonyl, benzenesulfonyl, toluenesulfonyl, cyano C1-C6 alkyl, (C1-C6 alkoxy)carbonyl(C1-C6 alkyl), (C1-C6 alkoxy)alkyl or -(C1-C6 alkyl)-S(O)_{d}(C1-C6 alkyl);
R⁴ is hydrogen, halogen, C1-C6 alkyl, C2-C6 alkenyl, C2-C6 alkynyl or cyano, wherein C1-C6 alkyl, C2-C6 alkenyl or C2-C6 alkynyl may be substituted with tri(C1-C6 alkyl)silyl on a terminal carbon atom;
a is any one of integers of 0 to 4;
R⁵ is selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino, di(C1-C6 alkyl)amino, (C1-C6 alkyl)carbonyl, (C1-C6 alkoxy) carbonyl, -C(O)-N(R^{A})₂, -S(O)_{d}-(C1-C6 alkyl), -SO₂-N(R^{A})₂, -N(R^{A})-C(O)-(C1-C6 alkyl), -N(R^{A})-C(O)-(halogen-substituted C1-C6 alkyl) and aryl;
wherein R^{A} in each occurrence independently represents hydrogen or C1-C6 alkyl;
wherein, optionally, aryl may be substituted with one or more substituents independently selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino and di(C1-C6 alkyl)amino;
b is 0 or 1;
c is 0 or 1;
R⁷ is selected from the group consisting of hydrogen, C1-C6 alkyl and tri(C1-C6 alkyl)silyl;
R² is selected from the group consisting of hydrogen, C1-C6 alkyl, halogen-substituted C1-C6 alkyl and -(CH₂)ₑ-Z-R⁶;
R³ is selected from the group consisting of C1-C6 alkyl, halogen-substituted C1-C6 alkyl and -(CH₂)ₑ-Z-R⁶;
wherein Z in each occurrence is independently selected from the group consisting of -S(O)_{d}-, -O-, -O-C(O)-, -NH- and -N(C1-C6 alkyl)-;
wherein R⁶ in each occurrence is independently selected from the group consisting of C1-C6 alkyl, halogen-substituted C1-C6 alkyl, C2-C6 alkenyl, aryl, aralkyl, biphenyl, C3-C7 cycloalkyl, C3-C7 cycloalkyl-(C1-C6 alkyl), heteroaryl and heteroaryl-(C1-C6 alkyl);
wherein a cycloalkyl, aryl or heteroaryl group, whether alone or as a part of a substituent group, may be substituted with one or more substituents independently selected from the group consisting of halogen, hydroxy, carboxy, C1-C6 alkyl, halogen-substituted C1-C6 alkyl, C1-C6 alkoxy, cyano, nitro, amino, C1-C6 alkylamino, di(C1-C6 alkyl)amino, -S(O)_{d}-(C1-C6 alkyl) and -SO₂-N(R^{A})₂;
provided that, when Z is O, NH or N(C1-C6 alkyl), R⁶ is selected from the group consisting of C1-C6 alkyl, halogen-substituted C1-C6 alkyl, aryl, aralkyl, biphenyl, C3-C7 cycloalkyl, C3-C7 cycloalkyl-(C1-C6 alkyl), heteroaryl and heteroaryl-(C1-C6 alkyl);
provided that, when R² is methyl, R³ is selected from the group consisting of C2-C6 alkyl, halogen-substituted C1-C6 alkyl and -(CH₂)ₑ-Z-R⁶;
provided that, when X is NR¹, R¹ is hydrogen or C1-C6 alkyl, b is 1, c is 0, R⁴ is hydrogen, R⁷ is hydrogen, a is 0 and when R² is CF₃, R³ is selected from the group consisting of C1-C6 alkyl, halogen-substituted C1-C6 alkyl except CF₃, and -(CH₂)ₑ-Z-R⁶;
provided that, when X is NH, R⁴ is methyl, b is 0, c is 0, R⁷ is hydrogen, a is 0 and when R² is methyl, R³ is selected from the group consisting of C1-C6 alkyl, halogen-substituted C1-C6 alkyl except CF₃, and -(CH₂)ₑ-Z-R⁶;
provided that, when X is NR¹, R¹ is hydrogen or C1-C6 alkyl, R⁴ is hydrogen or methyl, b is 0, c is 0, R⁷ is hydrogen, a is 0 and when R² is hydrogen or methyl, R³ is selected from the group consisting of C1-C6 alkyl, halogen-substituted C1-C6 alkyl and -(CH₂)ₑ-Z-R⁶, excluding -(CH₂)_{f}-N(R^{A})-(C1-C6 alkyl) or -(CH₂)₃-N(R^{A})-(benzyl);
provided that, when X is NH, R⁴ is hydrogen, b is 1, c is 0, R⁷ is hydrogen, a is 0 and when R² is hydrogen, R³ is selected from the group consisting of C1-C6 alkyl, halogen-substituted C1-C6 alkyl and -(CH₂)ₑ-Z-R⁶, excluding -(CH₂)-NH-(C1-C6 alkyl),
provided that, when X is NH, R⁴ is hydrogen, a is 0, R⁷ is hydrogen, b is 0, c is 0 and when R² is CF₃, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NH, R⁴ is hydrogen, a is 0, R⁷ is hydrogen, b is 1, c is 1 and when R² is hydrogen, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NR¹, R¹ is hydrogen or methyl, R⁴ is hydrogen or methyl, b is 0, c is 0, a is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding -CH₂-O-C1-C6 alkyl or -CH₂-O-benzyl;
provided that, when X is O, R⁴ is hydrogen, b is 0, c is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding CH₂-O-phenyl;
wherein, optionally, phenyl may be independently substituted with one to two substituents selected from among C1-C6 alkyl, hydroxy-substituted C1-C6alkyl, carboxy and -C(O)-(C1-C6 alkoxy);
d is any one of integers of 0 to 2;
e is any one of integers of 1 to 4; and
f is 1 or 2.

According to the present invention, it is possible to provide a compound that inhibits Aβ accumulation, and so on.

### Brief Description of the Drawings

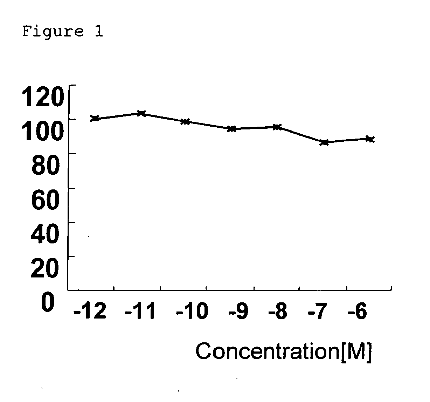

Figure 1 is a diagram showing results of measuring fluctuation caused by the test substance of the formula (Ia) in the amount of Aβ accumulation in cultured nerve cells (CHP212 cells).

In the diagram, each column represents, starting from the left, a measurement of Aβ accumulation amount: in a well to which the test substance has been added so that its final concentration can be 10⁻¹² M (10⁻¹² M), in a well to which the test substance has been added so that its final concentration can be 10⁻¹¹ M (10⁻¹¹ M), in a well to which the test substance has been added so that its final concentration can be 10⁻¹⁰ M (10⁻¹⁰ M), in a well to which the test substance has been added so that its final concentration can be 10⁻⁹ M (10⁻⁹ M), in a well to which the test substance has been added so that its final concentration can be 10⁻⁸ M (10⁻⁸ M), in a well to which the test substance has been added so that its final concentration can be 10⁻⁷ M (10⁻⁷ M), and in a well to which the test substance has been added so that its final concentration can be 10⁻⁶ M (10⁻⁶ M); in % by setting a measurement of Aβ accumulation amount in a control well to which only a solvent (DMSO) has been added as 100%.

### Mode for Carrying Out the Invention

The present invention will be described in detail below. A compound contained as an active ingredient (hereinafter, sometimes referred to as the present compound) in the pharmaceutical composition of the present invention (hereinafter, sometimes referred to as the present pharmaceutical composition) are described in, for example, Bioorganic & Medicinal Chemistry Letters, 2007, 17, 1784 and thus structures, production methods, etc. thereof are publicly known.

In the present invention, "alkyl" refers to a straight or branched-chain aliphatic saturated hydrocarbon group, and specific examples thereof include methyl, ethyl, propyl, isopropyl, isobutyl, n-butyl, tert-butyl, isopentyl and n-pentyl.

Examples of the C1-C6 alkyl preferably include methyl, ethyl, propyl and isopropyl.

As used in the present specification, the preferred number of atoms, such as carbon atoms, will be represented by, for example, the phrase "Cₓ-C_{y} alkyl", which refers to an alkyl group, as herein defined, containing the specified number of carbon atoms. Similar terminology will apply for other preferred terms and ranges as well.

In the present invention, "alkenyl" refers to a straight or branched-chain aliphatic unsaturated hydrocarbon group containing one or more carbon-to-carbon double bonds, and specific examples thereof include vinyl, isopropenyl and 3-methyl-2-butenyl.

Examples of the C2-C6 alkenyl preferably include vinyl, propenyl and propa-1,2-dienyl.

In the present invention, "alkynyl" refers to a straight or branched-chain aliphatic unsaturated hydrocarbon group containng one or more carbon-to-carbon triple bonds, and specific examples thereof include ethynyl, 2-propynyl and propargyl.

Examples of the C2-C6 alkynyl preferably include propynyl and propargyl.

In the present invention, "cycloalkyl" refers to a monocyclic or polycyclic aliphatic saturated hydrocarbon group, and specific examples thereof include cyclopropyl, cyclobutyl, cyclopentyl, cyclohexyl and cycloheptyl.

Examples of the C3-C7 cycloalkyl preferably include cyclopropyl.

In the present invention, "aryl" refers to a group forming an aromatic hydrocarbon ring, may be optionally substituted and may be fused to one or more of aryl ring(s) or cycloalkyl ring(s). Specific examples of the ring include benzene, anthracene, phenanthrene, naphthalene and biphenyl.

In the present invention, "heteroaryl" refers to a group forming an aromatic ring containing one or more heteroatoms, and may be optionally substituted. Examples of the heteroatoms include a nitrogen atom, an oxygen atom and a sulfur atom, including N-oxide, sulfur oxide and dioxide, and may be fused to one or more of heteroaryl ring(s), aryl ring(s), or cycloalkyl ring(s). Specific examples of the ring include furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole and indazole.

In the present invention, "alkoxy" refers to a group -OR, where R denotes C1-C6 alkyl.

Examples of the C1-C6 alkoxy preferably include methoxy and ethoxy.

In the present invention, "halogen" refers to fluorine, chlorine, bromine or iodine.

In the present invention, "haloalkyl" refers to a branched or straight-chain saturated hydrocarbon group substituted with at least one halogen, and specific examples thereof include trifluoromethyl and 2,2,2-trifluoroethyl.

In the present invention, "aralkyl" means a lower alkyl group substituted with an aryl group, and specific examples thereof include benzyl group, phenylethyl group, phenylpropyl group, naphthylmethyl group and naphthyl ethyl group.

The compounds of formula (I) may crystallize in two or more forms, a characteristic known as polymorphism, and such polymorphic forms ("polymorphs") are within the scope of formula (I). Polymorphism generally can occur as a response to changes in temperature, pressure, or both. Polymorphism can also result from variations in the crystallization process. Polymorphs can be distinguished by various physical characteristics known in the art such as x-ray diffraction patterns, solubility, and melting point.

Certain of the compounds described herein contain one or more chiral centers, or may otherwise be capable of existing as multiple stereoisomers. The scope of the present invention includes mixtures of stereoisomers as well as purified enantiomers or enantiomerically/diastereomerically enriched mixtures. Also included within the scope of the invention are the individual isomers of the compounds of formula (I), as well as any wholly or partially equilibrated mixtures thereof. The present invention also includes the individual isomers of the compounds of the formula above as mixtures with isomers thereof in which one or more chiral centers are inverted.

Examples of a preferred aspect of the present compound include the following compounds:
3-(5,6-dichloro-1H-benzoimidazol-2-yl)-3-hydroxy-butyronitrile ,
2-(5,6-dichloro-1-ethyl-1H-benzoimidazol-2-yl)-1-(2,2,2-triflu oro-ethylsulfanyl)-propan-2-ol,
2-(5,6-dichloro-1-methyl-1H-benzoimidazol-2-yl)-1-phenylmethan esulfonyl-propan-2-ol,
2-(5,6-dichloro-1-ethyl-1H-benzoimidazol-2-yl)-1-(4-methoxy-ph enylmethanesulfonyl)-propan-2-ol,
2-(5,6-dichloro-1-ethyl-1H-benzoimidazol-2-yl)-1-ethylsulfanyl -propan-2-ol,
2-(5,6-dichloro-1-ethyl-1H-benzoimidazol-2-yl)-1-(2,2,2-triflu oro-ethylsulfonyl)-propan-2-ol,
2-(5,6-dichloro-1-ethyl-1H-benzoimidazol-2-yl)-1-ethanesulfony 1-propan-2-ol,
1-chloro-2-(5,6-dichloro-1-methyl-1H-benzoimidazol-2-yl)-propa n-2-ol,
2-(5,6-dichloro-1-methyl-1H-benzoimidazol-2-yl)-1-isobutylsulf anyl-propan-2-ol,
2-(5,6-dichloro-1-methyl-1H-benzoimidazol-2-yl)-1-(2-methyl-pr opane-1-sulfonyl)-propan-2-ol,
2-(5,6-dichloro-1-methyl-1H-benzoimidazol-2-yl)-1-ethylsulfany 1-propan-propan-2-ol,
N-{4-[2-(5,6-dichloro-1-methyl-1H-benzoimidazol-2-yl)-2-hydrox y-propylsulfanyl]-phenyl}-acetamide,
5,6-dichloro-2-(2,2,2-trifluoro-ethyl)-1H-benzoimidazole,
2-[5,6-dichloro-2-(2,2,2-trifluoro-ethyl)-benzoimidazol-1-yl]-1-phenyl-ethanone,
2-[5,6-dichloro-2-(2,2,2-trifluoro-ethyl)-benzoimidazol-1-yl]-1-(4-fluoro-phenyl)-ethanone,
2-[5,6-dichloro-2-(2,2,2-trifluoro-ethyl)-benzoimidazol-1-yl]-1-(4-nitro-phenyl)-ethanone,
2-[5,6-dichloro-2-(2,2,2-trifluoro-ethyl)-benzoimidazol-1-yl]-1-(2,4-dimethoxy-phenyl)-ethanone,
5,6-dichloro-1-methyl-2-(2,2,2-trifluoro-ethyl)-1H-benzoimidaz ole,
[5,6-dichloro-2-(2,2,2-trifluoro-ethyl)-benzoimidazol-1-yl]-ac etic acid ethyl ester
1-benzofuran-2-yl-2-[5,6-dichloro-2-(2,2,2-trifluoro-ethyl)-be nzoimidazol-1-yl]-ethanone, (20)
5,6-dichloro-1-(4-fluoro-benzyl)-2-(2,2,2-trifluoro-ethyl)-1H-benzoimidazole,
5,6-dichloro-2-(2,2,2-trifluoro-ethyl)-1-(3-trifluoromethyl-be nzyl)-1H-benzoimidazole,
5,6-dichloro-1-pentafluorophenylmethyl-2-(2,2,2-trifluoro-ethy 1)-1H-benzoimidazole,
5,6-dichloro-1-(3-methyl-benzyl)-2-(2,2,2-trifluoro-ethyl)-1H-benzoimidazole,
5,6-dichloro-1-pyridin-3-ylmethyl-2-(2,2,2-trifluoro-ethyl)-1H -benzoimidazole,
5,6-dichloro-1-(4-nitro-benzyl)-2-(2,2,2-trifluoro-ethyl)-1H-b enzoimidazole,
2-[5,6-dichloro-2-(2,2,2-trifluoro-ethyl)-benzoimidazol-1-yl]-ethanol,
5,6-dichloro-1-[2-(4-fluoro-phenoxy)-ethyl]-2-(2,2,2-trifluoro -ethyl)-1H-benzoimidazole,
5,6-dichloro-1-[2-(3-fluoro-phenoxy)-ethyl]-2-(2,2,2-trifluoro -ethyl)-1H-benzoimidazole,
5,6-dichloro-1-[2-(4-chloro-phenoxy)-ethyl]-2-(2,2,2-trifluoro -ethyl)-1H-benzoimidazole,
5,6-dichloro-1-(3-methoxy-benzyl)-2-(2,2,2-trifluoro-ethyl)-1H -benzoimidazole,
5,6-dichloro-1-(2-methoxy-5-nitro-benzyl)-2-(2,2,2-trifluoro-e thyl)-1H-benzoimidazole,
4-{2-[5,6-dichloro-2-(2,2,2-trifluoro-ethyl)-benzoimidazol-1-y 1]-ethoxy}-benzonitrile,
5,6-dichloro-2-(2,2,2-trifluoro-ethyl)-1-(3-trifluoromethoxy-b enzyl)-1H-benzoimidazole,
5,6-dichloro-2-(2,2,2-trifluoro-ethyl)-1-(4-trifluoromethylsul fanyl-benzyl)-1H-benzoimidazole,
5,6-dichloro-1-(2-nitro-benzyl)-2-(2,2,2-trifluoro-ethyl)-1H-b enzoimidazole,
5,6-dichloro-1-(3-nitro-benzyl)-2-(2,2,2-trifluoro-ethyl)-1H-b enzoimidazole,
5,6-dichloro-1-(4-methanesulfonyl-benzyl)-2-(2,2,2-trifluoro-e thyl)-1H-benzoimidazole,
[5,6-dichloro-2-(2,2,2-trifluoro-ethyl)-benzoimidazol-1-yl]-ac etonitrile,
5,6-dichloro-1-(2-methyl-benzyl)-2-(2,2,2-trifluoro-ethyl)-1H-benzoimidazole,
5,6-dichloro-2-(2,2,2-trifluoro-ethyl)-1-(2-trifluoromethyl-be nzyl)-1H-benzoimidazole,
1-(2,4-bis-trifluoromethyl-benzyl)-5,6-dichloro-2-(2,2,2-trifl uoro-ethyl)-1H-benzoimidazole,
1-(2-benzenesulfonyl
methyl-benzyl)-5,6-dichloro-2-(2,2,2-trifluoro-ethyl)-1H-benzo imidazole,
1-biphenyl-2-ylmethyl-5,6-dichloro-2-(2,2,2-trifluoro-ethyl)-1 H-benzoimidazole,
5,6-dichloro-1-methoxymethyl-2-(2,2,2-trifluoro-ethyl)-1H-benz oimidazole,
5,6-dichloro-1-methylsulfanylmethyl-2-(2,2,2-trifluoro-ethyl)-1H-benzoimidazole,
5,6-dichloro-1-methylsulfonyl
methyl-2-(2,2,2-trifluoro-ethyl)-1H-benzoimidazole,
1-(4-bromo-phenyl)-2-[5,6-dichloro-2-(2,2,2-trifluoro-ethyl)-b enzoimidazol-1-yl]-ethanone,
1-[5,6-dichloro-2-(2,2,2-trifluoro-ethyl)-benzoimidazol-1-yl]-butan-2-one,
1-(4-chloro-phenyl)-2-[5,6-dichloro-2-(2,2,2-trifluoro-ethyl)-benzoimidazol-1-yl]-ethanone,
2-[5,6-dichloro-2-(2,2,2-trifluoro-ethyl)-benzoimidazol-1-yl]-1-(3-methoxy-phenyl)-ethanone,
2-[5,6-dichloro-2-(2,2,2-trifluoro-ethyl)-benzoimidazol-1-yl]-1-pyridin-3-yl-ethanone,
2-[5,6-dichloro-2-(2,2,2-trifluoro-ethyl)-benzoimidazol-1-yl]-1-(5-pyridin-2-yl-thiophen-2-yl)-ethanone,
2-[5,6-dichloro-2-(2,2,2-trifluoro-ethyl)-benzoimidazol-1-yl]-1-(2-methoxyphenyl)-ethanone,
2-[5,6-dichloro-2-(2,2,2-trifluoro-ethyl)-benzoimidazol-1-yl]-1-thiophen-2-yl-ethanone,
2-[5,6-dichloro-2-(2,2,2-trifluoro-ethyl)-benzoimidazol-1-yl]-1-pyridin-2-yl-ethanone,
2-[5,6-dichloro-2-(2,2,2-trifluoro-ethyl)-benzoimidazol-1-yl]-1-(3-nitro-phenyl)-ethanone,
2-[5,6-dichloro-2-(2,2,2-trifluoro-ethyl)-benzoimidazol-1-yl]-1-(4-nitro-phenyl)-ethanone,
1-benzyl-5,6-dichloro-2-(2,2,2-trifluoro-ethyl)-1H-benzoimidaz ole,
5,6-dichloro-2-(2,2,2-trifluoro-ethyl)-1-(4-trifluoromethyl-be nzyl)-1H-benzoimidazole,
5,6-dichloro-2-(2,2,2-trifluoro-ethyl)-1-(4-trifluoromethoxy-b enzyl)-1H-benzoimidazole,
5,6-dichloro-1-pyridin-2-ylmethyl-2-(2,2,2-trifluoro-ethyl)-1H -benzoimidazole,
2-[5,6-dichloro-2-(2,2,2-trifluoro-ethyl)-benzoimidazol-1-yl]-1-(2,3-dihydrobenzo[1,4]dioxin-6-yl)-ethanone,
5,6-dichloro-1-pyridin-4-ylmethyl-2-(2,2,2-trifluoro-ethyl)-1H -benzoimidazole,
3-[5,6-dichloro-2-(2,2,2-trifluoro-ethyl)-benzoimidazol-1-ylme thyl]-benzonitrile,
2-[5,6-dichloro-2-(2,2,2-trifluoro-ethyl)-benzoimidazol-1-ylme thyl]-benzonitrile,
2-[5,6-dichloro-2-(2,2,2-trifluoro-ethyl)-benzoimidazol-1-yl]-1-(5-methyl-3-phenyl-isoxazol-4-yl)-ethanone,
4-[5,6-dichloro-2-(2,2,2-trifluoro-ethyl)-benzoimidazol-1-ylme thyl]-benzonitrile,
5,6-dichloro-1-(2-fluoro-benzyl)-2-(2,2,2-trifluoro-ethyl)-1H-benzoimidazole,
5,6-dichloro-1-(3-fluoro-benzyl)-2-(2_{;}2,2-trifluoro-ethyl)-1H-benzoimidazole,
5,6-dichloro-1-(3-chloro-benzyl)-2-(2,2,2-trifluoro-ethyl)-1H-benzoimidazole,
5,6-dichloro-1-(4-chloro-benzyl)-2-(2,2,2-trifluoro-ethyl)-1H-benzoimidazole,
5,6-dichloro-1-(2-chloro-benzyl)-2-(2,2,2-trifluoro-ethyl)-1H-benzoimidazole,
5,6-dichloro-1-(4-pyrazol-1-yl-benzyl)-2-(2,2,2-trifluoro-ethy 1)-1H-benzoimidazole,
5,6-dichloro-1-(4-[1,2,3]thiadiazol-1-yl-benzyl)-2-(2,2,2-trif luoro-ethyl)-1H-benzoimidazole,
5,6-dichloro-1-(5-methyl-isoxazol-4-ylmethyl)-2-(2,2,2-trifluo ro-ethyl)-1H-benzoimidazole,
5,6-dichloro-1-(4-pyrrol-1-yl-benzyl)-2-(2,2,2-trifluoro-ethyl )-1H-benzoimidazole,
1-[5,6-dichloro-2-(2,2,2-trifluoro-ethyl)-benzoimidazol-1-yl]-butan-2-one oxime,
1-(4-benzyloxy-benzyl)-5,6-dichloro-2-(2,2,2-trifluoro-ethyl)-1H-benzoimidazole,
5,6-dichloro-1-(4-methoxy-benzyl)-2-(2,2,2-trifluoro-ethyl)-1H -benzoimidazole,
1-[5,6-dichloro-2-(2,2,2-trifluoro-ethyl)-benzoimidazol-1-yl]-butan-2-ol,
1-(4-bromo-benzyl)-5,6-dichloro-2-(2,2,2-trifluoro-ethyl)-1H-b enzoimidazole,
4-[5,6-dichloro-2-(2,2,2-trifluoro-ethyl)-benzoimidazol-1-ylme thyl]-benzoic acid ethyl ester,
1-(2-bromo-benzyl)-5,6-dichloro-2-(2,2,2-trifluoro-ethyl)-1H-b enzoimidazole,
1-(5,6-dimethyl-1H-benzoimidazol-2-yl-)2,2,2-trifluoro-ethanol ,
1-(5-chloro-6-fluoro-1H-benzoimidazol-2-yl-)2,2,2-trifluoro-et hanol,
1-(5-chloro-6-fluoro-1H-benzoimidazol-2-yl-)2,2,2-trifluoro-et hanone
1-(5,6-difluoro-1H-benzoimidazol-2-yl-)2,2,2-trifluoro-ethanol ,
1-(5,6-dichloro-1H-benzoimidazol-2-yl-)2,2,2-trifluoro-ethanol ,
diastereomers of 3,3,3-trifluoro-2-methoxy-2-phenyl-propionic acid
1-(5,6-dichloro-1H-benzoimidazol-2-yl)-2,2,2-trifluoro-1-methy 1-ethyl ester,
1-(5,6-difluoro-1H-benzoimidazol-2-yl-)2,2,2-trifluoro-ethane, 1-(5,6-difluoro-1H-benzoimidazol-2-yl-)2,2,2-trifluoro-ethane oxime,
1-(5-chloro-6-methyl-1H-benzoimidazol-2-yl-)2,2,2-trifluoro-et hanol,
1-(5-chloro-6-methyl-1H-benzoimidazol-2-yl-)2,2,2-trifluoro-et hanone,
1-(5-chloro-1H-benzoimidazol-2-yl-)2,2,2-trifluoro-ethanol, 2-(2,2,2-trifluoro-1-hydroxy-ethyl)-6-trifluoromethyl-1H-benzo imidazole-5-carbonitrile,
1-(5,6-dinitro-1H-benzoimidazol-2-yl-)2,2,2-trifluoro-ethanol, 1-(5,6-dimethoxy-1H-benzoimidazol-2-yl-)2,2,2-trifluoro-ethano 1,
3-(5,6-dichloro-2-1H-benzoimidazol-2-yl)-1,1,1-trifluoro-2-met hyl-propan-2-ol,
5,6-dichloro-2-(1,2,2,2-tetrafluoro-ethyl)-1H-benzoimidazole, 5,6-dichloro-2-(pentafluoroethyl)-1H-benzoimidazole,
4-[5,6-dichloro-2-(2,2,2-trifluoro-ethyl)-benzoimidazol-1-ylme thyl]-benzaldehyde,
(+)-1-(5,6-dichloro-1H-benzoimidazol-2-yl-)2,2,2-trifluoro-eth anol
(-)-1-(5,6-dichloro-1H-benzoimidazol-2-yl-)2,2,2-trifluoro-eth anol
5,6-dichloro-2-(2,2,2-trifluoro-ethyl)-1H-benzoimidazole,
1-(5,6-dichloro-1H-benzoimidazol-2-yl)-1-(2,2,2-trifluoromethy 1)-butanol,
1-(3,4-dichloro-phenylsulfanyl)-2-(5-nitro-6-trifluoromethyl-1 H-indol-2-yl)-propan-2-ol,
1-(4-chloro-phenylsulfanyl)-2-(1H-indol-2-yl)-propan-2-ol, 2-(5-chloro-6-trifluoromethyl-1H-indol-2-yl)-1-(4-fluoro-benze nesulfonyl)-propan-2-ol,
1-ethanesulfinyl-2-(5-nitro-6-trifluoromethyl-1H-indol-2-yl)-p ropan-2-ol,
1-ethanesulfonyl-2-(5-nitro-6-trifluoromethyl-1H-indol-2-yl)-p ropan-2-ol,
1-ethylsulfanyl-2-(5-fluoro-6-trifluoromethyl-1H-indol-2-yl)-p ropan-2-ol,
2-(5-amino-6-trifluoromethyl-1H-indol-2-yl)-1H-ethylsulfanyl-p ropan-2-ol,
2-(2-ethylsulfanyl-1-methoxy-1-methyl-ethyl)-5-nitro-6-trifluo romethyl-1H-indole,
2-ethanesulfinyl-1-(5-nitro-6-trifluoromethyl-1H-indol-2-yl)-e thanol,
2-ethylsulfanyl-1-(5-nitro-6-trifluoromethyl-1H-indol-2-yl)-et hanol,
2-(5-nitro-6-trifluoromethyl-1H-indol-2-yl)-1-(2,2,2-trifluoro -ethylsulfanyl)-butan-2-ol,
2-(5-chloro-6-trifluoromethyl-1H-indol-2-yl)-1-(2,2,2-trifluor o-ethylsulfanyl)-butan-2-ol,
2-(3-chloro-5-nitro-6-trifluoromethyl-1H-indol-2-yl)-1-(2,2,2-trifluoro-ethanesulfonyl)-butan-2-ol,
2-(5-nitro-6-trifluoromethyl-1H-indol-2-yl)-1-(2,2,2-trifluoro -ethanesulfonyl)-butan-2-ol,
2-(5-fluoro-6-trifluoromethyl-1H-indol-2-yl)-1-(2,2,2-trifluor o-ethylsulfanyl)-butan-2-ol,
1-butylsulfanyl-2-(5-nitro-6-trifluoromethyl-1H-indol-2-yl)-pr opan-2-ol,
2-((6-chloro-5-fluoro-1H-indol-2-yl)-1-ethylsulfanyl-propan-2-ol,
2-(2-ethylsulfanyl-1-hydroxy-1-methyl-ethyl)-6-trifluoromethyl -1H-indole-5-carbonitrile,
1-benzylsulfanyl-2-(5-nitro-6-trifluoromethyl-1H-indol-2-yl)-p ropan-2-ol,
1-isopropylsulfanyl-2-(5-nitro-6-trifluoromethyl-1H-indol-2-yl )-propan-2-ol,
2-(5-nitro-6-trifluoromethyl-1H-indol-2-yl)-1-(2,2,2-trifluoro -ethylsulfanyl)-propan-2-ol,
1-cyclopentylsulfanyl-2-(5-nitro-6-trifluoromethyl-1H-indol-2-yl)-propan-2-ol,
2-(5-nitro-6-trifluoromethyl-1H-indol-2-yl)-1-propylsulfanyl-p ropan-2-ol,
1-ethylsulfanyl-2-(5-nitro-6-trifluoromethyl-1H-indol-2-yl)-bu tan-2-ol,
4-methylsulfanyl-2-(5-nitro-6-trifluoromethyl-1H-indol-2-yl)-b utan-2-ol,
2-(5-nitro-6-trifluoromethyl-1H-indol-2-yl)-1-(propane-1-sulfo nyl)-propan-2-ol,
4-methanesulfonyl-2-(5-nitro-6-trifluoromethyl-1H-indol-2-yl)-butan-2-ol,
4-methanesulfinyl-2-(5-nitro-6-trifluoromethyl-1H-indol-2-yl)-butan-2-ol,
1-ethanesulfonyl-2-(5-nitro-6-trifluoromethyl-1H-indol-2-yl)-b utan-2-ol,
2-(5-nitro-6-trifluoromethyl-1H-indol-2-yl)-1-(2,2,2-trifluoro -ethanesulfonyl)-propan-2-ol,
2-(5-nitro-6-trifluoromethyl-1H-indol-2-yl)-1-(2,2,2-trifluoro -ethanesulfinyl)-propan-2-ol,
2-(5-nitro-6-trifluoromethyl-1H-indol-2-yl)-1-phenylmethanesul fonyl-propan-2-ol,
2-(5-nitro-6-trifluoromethyl-1H-indol-2-yl)-1-phenylmethanesul finyl-propan-2-ol,
5-chloro-2-isopropenyl-1-methanesulfonyl-6-trifluoromethyl-1H-indole,
5-chloro-1-methanesulfonyl-2-(2-methyl-oxiranyl)-6-trifluorome thyl-1-indole,
5-chloro-1-methanesulfonyl-6-trifluoromethyl-1H-indol-2-yl)-et hanone
2-(5-chloro-6-trifluoromethyl-1H-indol-2-yl)-1-(4-fluoro-pheny lsulfanyl)-propan-2-ol,
2-isopropenyl-1-methanesulfonyl-5-nitro-6-trifluoromethyl-1H-i ndole,
1-methanesulfonyl-2-(2-methyl-oxiranyl)-5-nitro-6-trifluoromet hyl-1H-indole,
1-(1-methanesulfonyl-5-nitro-6-trifluoromethyl-1H-indol-2-yl)-ethanone,
1-isobutylsulfanyl-2-(5-nitro-6-trifluoromethyl-1H-indol-2-yl) -propan-2-ol,
2-(2-ethylsulfanyl-1-hydroxy-1-methyl-ethyl)-3-methyl-6-triflu oromethyl-1H-indole-5-carbonitrile,
2-(5-bromo-3-methyl-6-trifluoromethyl-1H-indol-2-yl)-1-ethylsu lfanyl-propan-2-ol,
1-ethylsulfanyl-2-(3-methyl-5-nitro-6-trifluoromethyl-1H-indol -2-yl)-propan-2-ol,
2-(2,2,2-trifluoro-1-hydroxy-1-methyl-ethyl)-6-trifluoromethyl -1H-indole-5-carbonitrile,
(+) and (-) enantiomers of 2-(2,2,2-trifluoro-1-hydroxy-1-methyl-ethyl)-6-trifluoromethyl -1H-indole-5-carbonitrile,
(+) and (-) enantiomers of 2-(2-ethylsulfanyl-1-hydroxy-1-methyl-ethyl)-6-trifluoromethyl -1H-indole-5-carbonitrile,
(-) enantiomer of 2-(2-ethanesulfonyl-1-hydroxy-1-methyl-ethyl)-6-trifluoromethy l-1H-indole-5-carbonitrile,
(+) and (-) enantiomers of 2-(2-ethylsulfanyl-1-hydroxy-1-methyl-ethyl)-3-methyl-6-triflu oromethyl-1H-indole-5-carbonitrile,
(-) enantiomer of 2-(2-ethanesulfonyl-1-hydroxy-1-methyllethyl)-3-methyl-6-trifl uoromethyl-1H-indole-5-carbonitrile,
2-(1,2-dihydroxy-1-methyl-ethyl)-6-trifluoromethyl-1H-indole-5 -carbonitrile,
2-[2-(4-cyano-phenoxy)-1-hydroxy-1-methyl-ethyl]-6-trifluorome thyl-1H-indole-5-carbonitrile, and
2-[2-(4-cyano-3-trifluoromethyl-phenoxy)-1₋hydroxy-1-methyl-et hyl]-6-trifluoromethyl-1H-indole-5-carbonitrile.

The method of producing the present compound will be described below.

Abbreviations used in the description of the production method are as follows.
DABCO = 1,4-diazabicyclo[2.2.2]octane
DIPEA or DIEA or iPr₂NEt = diisopropylethylamine
DMAC = N,N-dimethylacetamide
DMF = N,N-dimethylformamide
DMSO = dimethyl sulfoxide
NBS = N-bromosuccinimide
NIS = N-iodosuccinimide
NMP = 1-methyl-2-pyrrolidinone
PdCl₂(PPh₃)₂ = bis(triphenylphosphine)palladium(II) chloride Pd₂(dba)₃ =
tris[µ-(1,2-η:4,5-η)-(1E,4E)-1,5-diphenyl-1,4-pentadien-3-one] ]dipalladium
PdCl₂(dppf) = 1,1'-bis(diphenylphosphino)ferrocenepalladium chloride
Pd(OAc)₂ = palladium(II) acetate
Ph₃ P = triphenylphosphine
TBAF = tetrabutylammonium fluoride
THF = tetrahydrofuran

The present compound of the following formula (I): 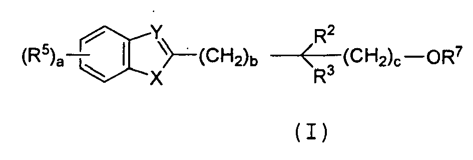 can be prepared according to the method shown in the following scheme.

The compound of the formula (I) (b is 0, c is 0, Y is CH and X is NR¹) can be prepared according to the method summarized in the following scheme 1.

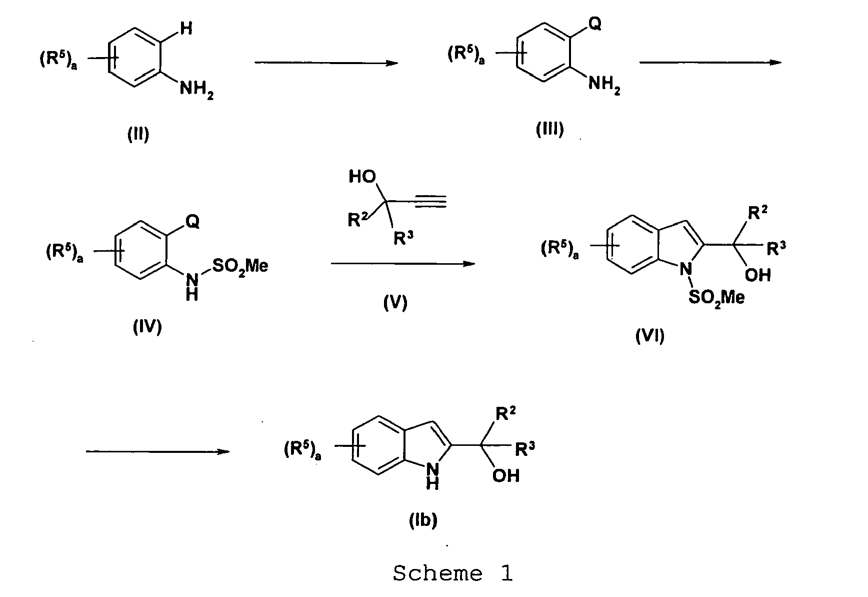

It is possible to produce the compound of the formula (III) wherein Q is I or Br by reacting a known compound, or a properly substituted compound of the compound of the formula (II) prepared by a known method with an iodine or bromine source (for example, NIS, ICI, NBS, Br₂ and I₂), optionally in the presence of a catalyst [for example, acetic acid (together with ICI), and toluenesulfonic acid (together with NIS or NBS)], in an organic solvent or a mixture thereof (for example, THF, methanol, acetic acid and THF/methanol).

The compound of the formula (IV) can be produced by reacting the compound of the formula (III) with mesyl chloride (or p-toluenesulfonyl chloride) in an organic solvent (for example, THF, pyridine and DMF) in the presence of an organic base (for example, pyridine and potassium t-butoxide).

The compound of the formula (VI) can be produced by reacting the compound of the formula (IV) with a known compound, or a properly substituted compound of the compound of the formula (V) prepared by a known method in an organic solvent (for example, THF, DMF and DMAC) in the presence of an organic base, preferably a tertiary amine base (for example, TEA, DIPEA and pyridine) , CuI and a catalyst (for example, PdCl₂ (PPh₃)₂, Pd₂(dba)₃ and PdCl₂ (dppf) ) .

The compound of the formula (Ib) can be produced by reacting the compound of the formula (VI), for example, optionally with a base (for example, NaOH, KOH and NaO (lower alkyl)) in an organic solvent or a mixture thereof (for example, methanol/water, ethanol/water and THF) and deprotecting the resultant according to a known method.

Alternately, the compound of the formula (Ib) can be produced by reacting the compound of the formula (VI) with TBAF at a high temperature of preferably about 50°C or higher in an organic solvent (for example, THF and DMF).

The compound of the formula (I) (b is 0, c is 0, Y is CR⁴ and X is NR¹, and R⁴ is lower alkyl, and more preferably methyl) can be prepared according to the method shown in Scheme 2.

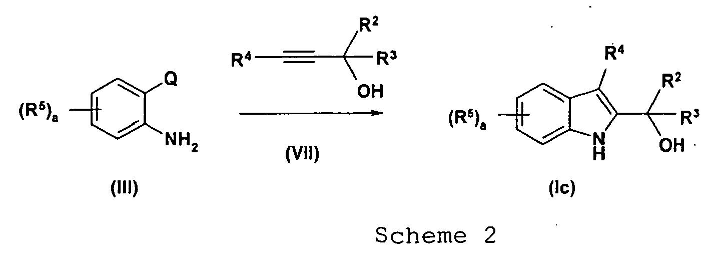

The compound of the formula (Ic) can be produced by reacting a properly substituted compound of the compound of the formula (III) with a known compound, or a properly substituted compound of the compound of the formula (VII) prepared by a known method at a high temperature of preferably about 70°C or higher, and more preferably about 80°C in the presence of a base (for example, potassium acetate and DABCO) and a catalyst (for example, Pd(OAc)₂).

The compound of the formula (I) (c is 0, Y is N and X is NR¹) can be prepared according to the method shown in Scheme 3.

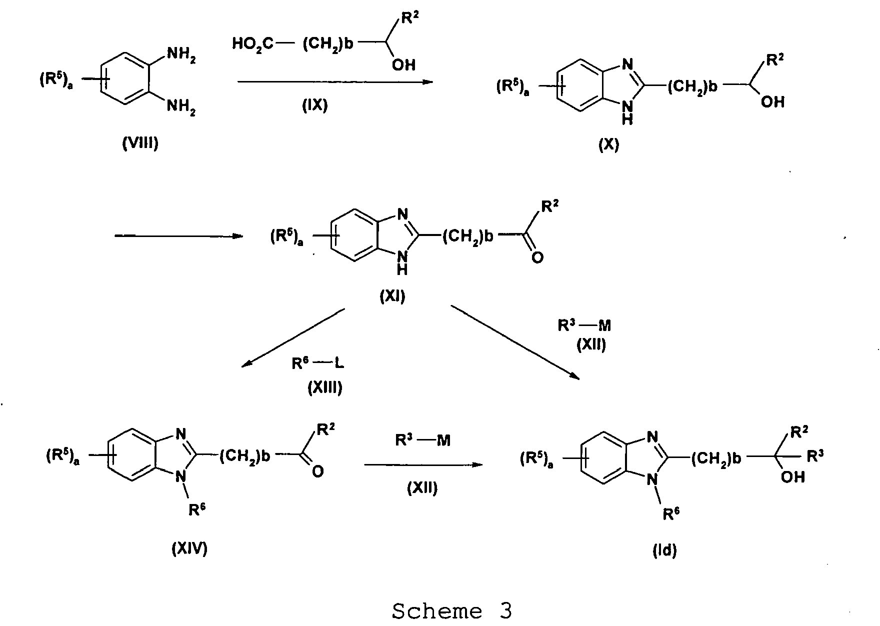

The compound of the formula (X) can be produced by reacting a known compound, or a properly substituted compound of the compound of the formula (VIII) prepared by a known method with a known compound, or the compound of the formula (IX) prepared by a known method in water in the presence of an acid (for example, hydrochloric acid, sulfuric acid and acetic acid).

The compound of the formula (XI) can be produced by reacting the compound of the formula (X) with an oxidizing agent (for example, Na₂Cr₂O₇) in an aqueous solvent in the presence of an acid (for example, sulfuric acid).

When R² is hydrogen in the formula (IX), the compound of the formula (XI) can be produced by reacting a proper oxidizing agent (for example, MnO₂) in an organic solvent (for example, dichloromethane, dichloroethane, benzene and toluene) at a temperature from room temperature to 110°C, preferably room temperature, or by conducting Dess-Martin Periodinane in an organic solvent (for example, dichloromethane and dichloroethane) at a temperature from 0°C to room temperature, preferably room temperature, or by reacting a mixture of (a) TEMPO, (b) bleach and (c)KBr or NaBr under cooling at a temperature from about -40°C to room temperature, preferably from about -10°C to room temperature.

It is possible to produce the compound of the formula (Id) wherein R¹ is hydrogen by reacting the compound of the formula (XI) , which is a known compound wherein M is MgBr or Li or a compound prepared by a known method, with a properly substituted compound of the compound of the formula (XII).

The compound of the formula (XIV) can be produced by reacting the compound of the formula (XI), which is a known compound having a proper leaving group (for example, Br and I) or a compound prepared by a known method, with a properly substituted compound of the compound of the formula (XIII) in the presence of a base (for example, NaH, K₂CO₃ and Na₂CO₃) in an organic solvent (for example, DMF, DMAC, DMSO and NMP).

It is possible to produce the compound of the formula (Id) wherein R¹ is other than hydrogen by reacting the compound of the formula (XIV) with a known compound wherein M is MgBr or Li, or a properly substituted compound of the compound of the formula (XII) prepared by a known method.

The present pharmaceutical composition contains the present compound or a pharmaceutically acceptable salt thereof.

Examples of the pharmaceutically acceptable salt include a salt with a physiologically acceptable acid (such as an inorganic acid and an organic acid) or base (such as an alkaline metal) , and a physiologically acceptable acid addition salt is particularly preferred. Examples of such acid addition salt include a salt with an inorganic acid (such as hydrochloric acid, phosphoric acid, hydrobromic acid and sulfuric acid) and a salt with an organic acid (such as acetic acid, formic acid, propionic acid, fumaric acid, maleic acid, succinic acid, tartaric acid, citric acid, malic acid, oxalic acid, benzoic acid, methanesulfonic acid and benzenesulfonic acid).

The present pharmaceutical composition may be used to inhibit Aβ accumulation as an Aβ accumulation-inhibitory agent (hereinafter, sometimes referred to as the present Aβ accumulation-inhibitory agent).

Also, it is possible to inhibit Aβ accumulation by (step of) administering an effective amount of the present pharmaceutical composition to a mammal which may be diagnosed with an Aβ-related disease. For example, by administering orally or parenterally an effective amount of the present Aβ accumulation-inhibitory agent to a mammal such as a human which may be diagnosed with an Aβ-related disease, it is possible to inhibit Aβ accumulation in the mammal.

In the case of orally administering the present Aβ accumulation-inhibitory agent to a mammal which may be diagnosed with an Aβ-related disease, the present Aβ accumulation-inhibitory agent may be used in an ordinary dosage form such as a tablet (including a sugar-coated tablet and a film-coated tablet), a pill, a granule, a powder, a capsule (including a soft capsule), a syrup, an emulsion, and a suspension.

In the case of parenteral administration, the present Aβ accumulation-inhibitory agent may be used in an ordinary liquid dosage form such as an emulsion and a suspension. Examples of a method of the parenteral administration include a method of injection and a method of administering a suppository into the rectum.

For a method for administering the present Aβ accumulation-inhibitory agent, a conventional method of administration such as the aforementioned oral administration and the parenteral administration may be appropriately selected without any particular limitation, and it may be used singly or in combination.

An appropriate dosage form of the present Aβ accumulation-inhibitory agent may be produced by mixing the present compound or a pharmaceutically acceptable salt thereof with an acceptable ordinary carrier, excipient, binder, stabilizer, diluent, and so on. Further, when the present Aβ accumulation-inhibitory agent is used in the form of an injection, an acceptable buffer, solubilizer, isotonic agent, and so on may be added.

As the present Aβ accumulation-inhibitory agent thus obtained is low-toxic, and thus highly safe, it may be used (administered) to, for example, a mammal (for example, a human, a rat, a mouse, a guinea pig, a rabbit, a sheep, a pig, a cow, a horse, a cat, a dog and a monkey). The dosage varies depending on age, sex, weight and severity of a disease of a mammal to be administered, a kind and an administration form (dosage form) of the present Aβ accumulation-inhibitory agent, and so on. Usually, for example, in the case of orally administering to a human, it may be administered at about 0.01 mg to about 1 g as the amount of an active ingredient, preferably about 0.1 mg to 1 g as the amount of an active ingredient, more preferably about 1.0 mg to 200 mg as the amount of an active ingredient, and even more preferably about 1.0 mg to 50 mg per day as the amount of an active ingredient, to an adult (for example, weighing 60 kg). The daily dose may also be administered in several divided dosage.

Also, in the case of parenterally administering to a human, it may be administered by an intravenous injection at, as the amount of an active ingredient, about 0.01 mg to 30 mg, preferably about 0.1 mg to 20 mg, and more preferably about 1.0 mg to 10 mg per day, to an adult (for example, weighing 60 kg).

Examples of a disease to which the present Aβ accumulation-inhibitory agent may be applicable include such a disease as an Aβ-related disease (amyloidosis).

The Aβ-related disease is classified into "localized amyloidosis" in which accumulation (deposition) is caused in only some organs and tissues in a living body, and "systemic amyloidosis" in which accumulation is caused in various organs and tissues throughout the body.

Examples of localized amyloidosis include Alzheimer's disease, Down's syndrome, cerebrovascular amyloidosis, hereditary amyloidotic cerebral hemorrhage, mad cow disease (bovine spongiform encephalopathy, BSE), variant Creutzfeldt-Jakob disease (vCJD), Gerstmann-Straussler-Scheinker syndrome, transmissible spongiform encephalopathy, scrapie, heart disease (senile cardiac amyloidosis), endocrine tumor (thyroid cancer), adult-onset diabetes, cutaneous amyloidosis and localized nodular amyloidosis.

Examples of systemic amyloidosis include familial amyloid polyneuropathy (FAP), tuberculosis, Hansen's disease, familial Mediterranean fever, bronchiectasis, Muckle-wells syndrome, reactive AA amyloidosis, immunocytic amyloidosis, osteomyelitis and cardiac failure. Other examples include senile amyloidosis which develops non-hereditarily at an advanced age, dialysis amyloidosis caused by amyloid that results from alteration in protein irremovable by a dialysis membrane used in treatment of dialysis patients, and secondary amyloidosis caused by amyloid that is generated by cleavage of a protein expressed in rheumatism.

### Examples

The present invention will be described below in further detail by example, however, the present invention is not limited by it.

### Example 1 Measurement of fluctuation in the amount of Aβ accumulation in cultured nerve cells

Using a phenol red-free DMEM medium (manufactured by Invitrogen Corporation) to which charcoal dextran-treated FBS has been added so that its final concentration can be 10%, cultured nerve cells (human neuroblastoma CHP212, ATCC) were inoculated in a six-well plate (manufactured by BD Falcon) at approximately 2 × 10⁵ cells/well, and then cultured overnight. After the cultivation, DMSO or a test substance of the formula (Ia) dissolved in DMSO was added to the cells. The cells were then cultured, and a protease inhibitor (p1860, manufactured by Sigma-Aldrich Corporation) and DMSO were added to each well 48 hours after the addition of the test substance, followed by measuring the amount of Aβ in the medium by ELISA method (Human/Rat Amyloid β1-42 measuring kit, Code. No. 290-62601, Wako Pure Chemical Industries, Ltd.) The results were shown in Figure 1.

It was observed that the amount of Aβ accumulation was markedly decreased by addition of the test substance of the formula (Ia).

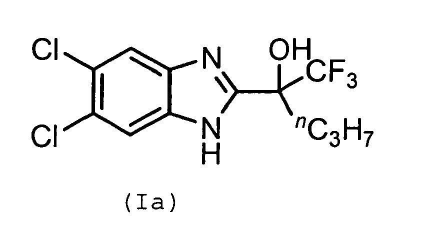

### Industrial Applicability

According to the present invention, it becomes possible to provide a compound that inhibits Aβ accumulation and so on.

## Claims

1. A pharmaceutical composition for inhibiting amyloid-β protein accumulation comprising as an active ingredient a compound of the following formula: 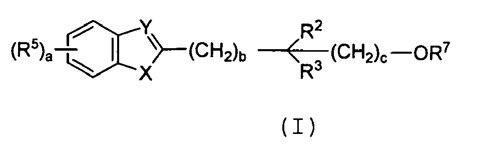 or a pharmaceutically acceptable salt thereof,
in the formula (I):
X is O, S or NR¹;
Y is N or CR⁴;
R¹ is hydrogen, C1-C6 alkyl, fluorinated C1-C6 alkyl, C1-C6 alkylsulfonyl, benzenesulfonyl, toluenesulfonyl, cyano C1-C6 alkyl, (C1-C6 alkoxy)carbonyl(C1-C6 alkyl), (C1-C6 alkoxy)alkyl or -(C1-C6 alkyl)-S(O)_{d}(C1-C6 alkyl);
R⁴ is hydrogen, halogen, C1-C6 alkyl, C2-C6 alkenyl, C2-C6 alkynyl or cyano, wherein C1-C6 alkyl, C2-C6 alkenyl or C2-C6 alkynyl may be substituted with tri(C1-C6 alkyl)silyl on a terminal carbon atom;
a is any one of integers of 0 to 4;
R⁵ is selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino, di(C1-C6 alkyl)amino, (C1-C6 alkyl)carbonyl, (C1-C6 alkoxy)carbonyl, -C(O)-N(R^{A})₂, -S(O)_{d}-(C1-C6 alkyl) , -SO₂-N(R^{A})₂, -N(R^{A})-C(O)-(C1-C6 alkyl), -N(R^{A})-C(O)-(halogen-substituted C1-C6 alkyl) and aryl;
wherein R^{A} in each occurrence independently represents hydrogen or C1-C6 alkyl;
wherein, optionally, aryl may be substituted with one or more substituents independently selected from the group consisting of halogen, -OH, carboxy, C1-C6 alkyl, halogen-substituted C1-C6 alkyl, C1-C6 alkoxy, halogen-substituted C1-C6 alkoxy, cyano, nitro, amino, C1-C6 alkylamino and di(C1-C6 alkyl)amino;
b is 0 or 1;
c is 0 or 1;
R⁷ is selected from the group consisting of hydrogen, C1-C6 alkyl and tri(C1-C6 alkyl)silyl;
R² is selected from the group consisting of hydrogen, C1-C6 alkyl, halogen-substituted C1-C6 alkyl and -(CH₂)ₑ-Z-R⁶;
R³ is selected from the group consisting of C1-C6 alkyl, halogen-substituted C1-C6 alkyl and -(CH₂)ₑ-Z-R⁶;
wherein Z in each occurrence is independently selected from the group consisting of -S(O)_{d}-, -O-, -O-C(O)-, -NH- and -N(C1-C6 alkyl)-;
wherein R⁶ in each occurrence is independently selected from the group consisting of C1-C6 alkyl, halogen-substituted C1-C6 alkyl, C2-C6 alkenyl, aryl, aralkyl, biphenyl, C3-C7 cycloalkyl, C3-C7 cycloalkyl-(C1-C6 alkyl), heteroaryl and heteroaryl-(C1-C6 alkyl);
wherein a cycloalkyl, aryl or heteroaryl group, whether alone or as a part of a substituent group, may be substituted with one or more substituents independently selected from the group consisting of halogen, hydroxy, carboxy, C1-C6 alkyl, halogen-substituted C1-C6 alkyl, C1-C6 alkoxy, cyano, nitro, amino, C1-C6 alkylamino, di(C1-C6 alkyl)amino, -S(O)_{d}-(C1-C6 alkyl) and -SO₂-N(R^{A})2;
provided that, when Z is O, NH or N(C1-C6 alkyl), R⁶ is selected from the group consisting of C1-C6alkyl, halogen-substituted C1-C6 alkyl, aryl, aralkyl, biphenyl, C3-C7 cycloalkyl, C3-C7 cycloalkyl-(C1-C6 alkyl), heteroaryl and heteroaryl-(C1-C6 alkyl);
provided that, when R² is methyl, R³ is selected from the group consisting of C2-C6 alkyl, halogen-substituted C1-C6 alkyl and - (CH₂)ₑ-Z-R⁶;
provided that, when X is NR¹, R¹ is hydrogen or C1-C6 alkyl, b is 1, c is 0, R⁴ is hydrogen, R⁷ is hydrogen, a is 0 and when R² is CF₃, R³ is selected from the group consisting of C1-C6 alkyl, halogen-substituted C1-C6 alkyl except CF₃, and -(CH₂)ₑ-Z-R⁶;
provided that, when X is NH, R⁴ is methyl, b is 0, c is 0, R⁷ is hydrogen, a is 0 and when R² is methyl, R³ is selected from the group consisting of C1-C6 alkyl, halogen-substituted C1-C6 alkyl except CF₃, and -(CH₂)ₑ-Z-R⁶;
provided that, when X is NR¹, R¹ is hydrogen or C1-C6 alkyl, R⁴ is hydrogen or methyl, b is 0, c is 0, R⁷ is hydrogen, a is 0 and when R² is hydrogen or methyl, R³ is selected from the group consisting of C1-C6 alkyl, halogen-substituted C1-C6 alkyl and -(CH₂ₑ-Z-R⁶, excluding -(CH₂)_{f}-N(R^{A})-(C1-C6 alkyl) or -(CH₂)₃-N(R^{A})-(benzyl);
provided that, when X is NH, R⁴ is hydrogen, b is 1, c is 0, R⁷ is hydrogen, a is 0 and when R² is hydrogen, R³ is selected from the group consisting of C1-C6 alkyl, halogen-substituted C1-C6 alkyl and -(CH₂)ₑ-Z-R⁶, excluding -(CH₂)-NH-(C1-C6 alkyl), provided that, when X is NH, R⁴ is hydrogen, a is 0, R⁷ is hydrogen, b is 0, c is 0 and when R² is CF₃, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NH, R⁴ is hydrogen, a is 0, R⁷ is hydrogen, b is 1, c is 1 and when R² is hydrogen, R³ is selected from the group consisting of C1-C6 alkyl, halogen-substituted C1-C6 alkyl and -(CH₂)ₑ-Z-R⁶, excluding -CH₂-O-C(O)-CH₃;
provided that, when X is NR¹, R¹ is hydrogen or methyl, R⁴ is hydrogen or methyl, b is 0, c is 0, a is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding -CH₂-O-C1-C6 alkyl or -CH₂-O-benzyl;
provided that, when X is O, R⁴ is hydrogen, b is 0, c is 0, R⁷ is hydrogen and when R² is hydrogen, R³ is selected from the group consisting of C1-C6 alkyl, halogen-substituted C1-C6 alkyl and -(CH₂)ₑ-Z-R⁶, excluding CH₂-O-phenyl;
wherein, optionally, phenyl may be independently substituted with one to two substituents selected from among C1-C6 alkyl,
hydroxy-substituted C1-C6 alkyl, carboxy and -C (O)-(C1-C6 alkoxy); d is any one of integers of 0 to 2;
e is any one of integers of 1 to 4; and
f is 1 or 2.

2. An amyloid-β protein accumulation-inhibitory agent comprising as an active ingredient the compound of formula (I) described in Claim 1 or a pharmaceutically acceptable salt thereof.

3. Use of the compound of formula (I) described in Claim 1 or a pharmaceutically acceptable salt thereof for inhibiting amyloid-β protein accumulation.

4. A method for inhibiting amyloid-β protein accumulation comprising administering an effective amount of the compound of formula (I) described in Claim 1 or a pharmaceutically acceptable salt thereof to a mammal which may be diagnosed with an amyloid-β protein-related disease.
